# EUROPÄISCHE PATENTSCHRIFT

(11) **EP 1 650 184 B1**
(45) Veröffentlichungstag und Bekanntmachung des Hinweises auf die Patenterteilung: **23.01.2008**
(21) Anmeldenummer: 05018346.6
(22) Anmeldetag: 24.08.2005
(51) Int. Cl.: C07C 213/06, C07C 213/04, C07C 215/16, C07C 217/50

(54) **Mischungen von Derivaten des N,N-Bis-(2-hydroxyalkyl)-4-toluidins, deren Herstellung und Verwendung**
Mixtures of N,N-Bis-(2-hydroxyalkyl)-4-toluidin, their preparation and use
Compositions de N,N-Bis-(2-hydroxyalkyl)-4-toluidin, leur procédé de fabrication et utilisation

(30) Priorität: 04.09.2004 DE 102004042858
(43) Veröffentlichungstag der Anmeldung: 26.04.2006
(73) Patentinhaber: Saltigo GmbH, 40764 Langenfeld (DE)
(72) Erfinder: Rauchschwalbe, Günter, Dr., 51375 Leverkusen (DE); Scheinert, Wolfgang, Dr., 51375 Leverkusen (DE)
(74) Vertreter: Wichmann, Birgid

(56) Entgegenhaltungen:
- RU-C1- 2 063 960

## Beschreibung

Die Erfindung betrifft neue Mischungen von Derivaten des N,N-Bis-(2-hydroxyalkyl)-4-toluidins, deren Herstellung sowie deren Verwendung als Polymerisationsbeschleuniger.

Die Einzelverbindung N,N-Bis-(2-hydroxyethyl)-4-toluidin ist bekannt. In J. Org. Chem. 26 (1961) 1477-1480 wird offenbart, dass sie durch Umsetzung von 4-Toluidin mit 2 mol Ethylenoxid hergestellt und dann z.B. durch Destillation oder Kristallisation gereinigt werden kann. N,N-Bis-(2-hydroxyethyl)-4-toluidin wird dabei in einer Ausbeute von 76% der Theorie erhalten.

Auch in Inorg. Chimica Acta 1995, 240, 257-62 ist die Herstellung von N,N-Bis-(2-hydroxyethyl)-4-substituierten Benzolen beschrieben. Zur Herstellung von N,N-Bis-(2-hydroxyethyl)-4-toluidin wird 4-Toluidin mit 2-Chlorethanol umgesetzt. Beschrieben wird ferner die Herstellung der Einzelverbindung N-(4-methylphenyl)-azatetraglykol 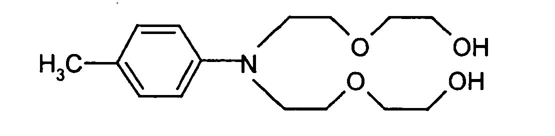 durch Umsetzung von 4-Toluidin mit 2-(2-Chloroethoxy)ethanol.

In der EP-A-1 256 615 werden zweikomponentige Klebemittelzusammensetzungen aus einer Klebemittelkomponente sowie einer Aktivatorkomponente offenbart. In der Klebemittelkomponente sind neben den Monomeren auf Acryl-/Methacrylbasis u.a. Reduktionsmittel enthalten, bei denen es sich um gegebenenfalls substituierte Aniline oder gegebenenfalls substituierte Toluidine handelt. Als substituierte Toluidine werden allgemein solche der Formel 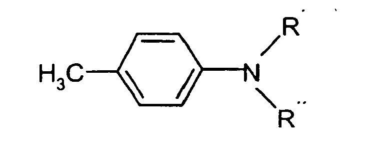 beschrieben, worin R' und R" gleich oder verschieden sein können und gemäß Verweis auf vorherige Definitionen u.a. CH₂CHY₂ bedeuten können, wobei Y unter vielen anderen Bedeutungen auch OCₙH₂ₙ₊₁ mit n kleiner 4 sein kann. Das einzige explizite Beispiel für ein entsprechend substituiertes para-Toluidin ist die in Example 4 erwähnte Einzelsubstanz N,N-bis (2-hydroxyethyl)-p-toluidin ("Emery 5710" von Cognis Corporation)

Aus WO-A-00/43425 ist es bekannt, dass N,N-bis-(2-hydroxyethyl)-4-toluidin als Beschleuniger für Polymerisationen oder Vulkanisationen eingesetzt werden kann. Beschrieben wird dort ein Redox-Initiator-System für die Herstellung von Polyester Granulaten durch Suspensions-Polymerisation, wobei dieses Redox-Initiator System ein Diacylperoxid und ein aromatisches Amin der Formel 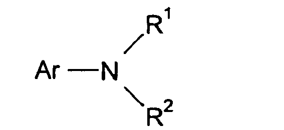 enthält, worin Ar eine gegebenenfalls substituierte Aryl-Gruppe ist und R¹ und R² allgemein definiert werden und unter anderem jeweils -(CHR'CHR'-O)ₙH bedeuten können mit n = 1-10 und R' gleich H oder C₁₋₃-Alkyl. Die Herstellung kommerziell nicht erhältlicher aromatischer Amine mit Polyoxyalkylen-Substituenten am Stickstoff durch Umsetzung der Hydroxyalkyl-Verbindung mit einem Alkylenoxid wird als dem Fachmann geläufig genannt. Einzig explizit genanntes und als bevorzugt bezeichnetes substituiertes para-Toluidin ist wiederum die Einzelsubstanz N,N-bis-(2-hydroxyethyl)-p-toluidin.

In EP-A-1 070 730 wird beschrieben, dass zur schnellen Aushärtung von Polymeren auf Methacrylat-Basis Beschleuniger zugesetzt werden können. Hierfür werden Kondensationsprodukte von para-Toluidin und Ethylenoxid genannt. Explizit beschrieben wird das Produkt "Bisomer PTE" (International Specialty Chemicals), für welches angegeben wird, dass zwei Ethylenoxid Einheiten an jeder der freien Valenzen des Stickstoffs sitzen.

Aus US 2003/0083443 A1 sind stabilisierte ungesättigte Polymer-Harz-Systeme bekannt, die ein tertiäres aromatisches Amin als Vulkanisationsbeschleuniger aufweisen. Die breite Definition des tertiären aromatischen Amins umfasst bei Kombination der Bedeutungen verschiedenster Substituenten auch tertiäre aromatische Amine mit Polyoxyalkylen-Substituenten mit 1-6 Oxyalkylen-Wiederholungseinheiten am Stickstoff. Als bevorzugte Vulkanisationsbeschleuniger werden jedoch solche genannt, die am Stickstoff 3 verschiedene Substituenten besitzen, wovon nur einer potentiell ein Polyoxyalkylen-Substituent ist (Paragraphen [0019] und [0026]). Einzig explizit in der Beschreibung genanntes substituiertes para-Toluidin ist auch hier wiederum die Einzelsubstanz N,N-bis-(2-hydroxyethyl)-p-toluidin (Paragraph [0075]). Ferner wird in Example 4 das Produkt der International Specialty Chemicals "Bisomer PTE" genannt.

Aus Chemia Stosowana 32, 3-4, 503-514 (1988**)** ist die Herstellung von Polyethoxylaten von 4-Alkylphenylaminen durch Reaktion von 4-Alkylphenylaminen mit Ethylenoxid in Gegenwart von Kaliumhydroxid bekannt und anhand der Beispiele von 4-Hexyl-, 4-Octyl-, 4-Decyl-, 4-Dodecyl- und 4-Hexadecylphenylamin untersucht.

RU 2 063 960 offenbart hydroxyethyliertes 4-Toluidin sowie ein Verfahren zur Herstellung und dessen Verwendung als Polymerisationsbeschleuniger. Bezüglich der Reinheit des 4-Toluidins ist nichts bekannt.

Die Aufgabe der vorliegenden Erfindung bestand darin, ein Verfahren bereitzustellen, mit dem weitere neue Derivate des N,N-Bis-(2-hydroxyalkyl)-4-toluidins erhalten werden können, die ebenfalls als Polymerisations- oder Vulkanisationsbeschleuniger eingesetzt werden können.

Gegenstand dieser Erfindung ist ein Verfahren zur Herstellung einer Mischung von zwei oder mehr verschiedenen Verbindungen der allgemeinen Formel (I), 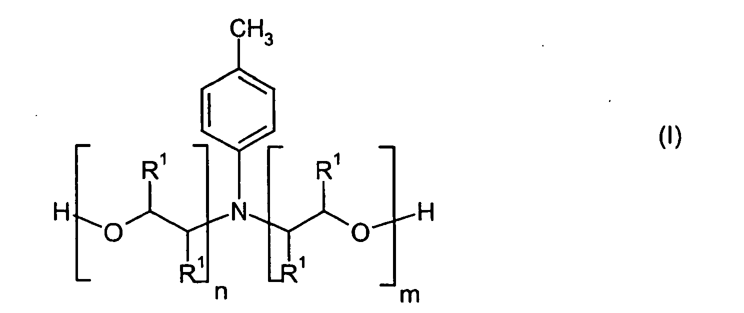 worin
- n und m: unabhängig voneinander jeweils eine ganze Zahl im Bereich von 0 bis 11 darstellen, die Summe aus n und m aber mindestens 1 beträgt und
- R¹: gleich oder verschieden sind und Wasserstoff oder Methyl bedeuten können, wobei aber zwei Reste R¹, die sich an direkt benachbarten Kohlenstoffatomen befinden, nicht gleichzeitig Methyl darstellen,
durch Umsetzung von 4-Toluidin, welches weniger als 0,2 Gew.% 3-Toluidin, bezogen auf 4-Toluidin, enthält, mit mindestens 2 mol eines Alkylenoxids der Formel (II) pro mol 4-Toluidin 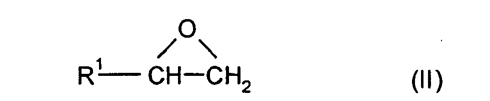 wobei R¹ die für die Formel (I) genannte Bedeutung besitzt.

Gegenstand der Erfindung ist auch eine nach diesem Verfahren erhältliche Mischung von zwei oder mehr verschiedenen Verbindungen der allgemeinen Formel (I).

Mit dem erfindungsgemäßen Verfahren gelingt es überraschenderweise, die Mischungen von Verbindungen der allgemeinen Formel (I) mit faktisch vollständigem Umsatz zu erhalten. Bei einer Nachweisgrenze per Gaschromatographie von 100 ppm kann in den Mischungen kein nicht-umgesetztes 4-Toluidin mehr nachgewiesen werden. Dies ist von wesentlicher Bedeutung, da die Toluidine starke Blutgifte und als krebserregend eingestuft sind. Die erhaltenen Mischungen sind exzellent als Polymerisations- oder Vulkanisationsbeschleuniger geeignet, bevorzugt in der radikalischen Polymerisation von Polyestern und insbesondere von ungesättigten Polyestern. Sie führen überraschenderweise zu keinerlei grünen oder grünlichen Verfärbungen des Polymers, so wie dies der Fall ist, wenn man ähnliche Mischungen einsetzt, die allerdings unter Verwendung von 4-Toluidin hergestellt wurden, welches einen Anteil von 0,2 Gew.% oder mehr 3-Toluidin enthielt. Im Normalfall besitzt das verfügbare 4-Toluidin einen Gehalt an 3-Toluidin von mindestens 0,2 Gew.%, da die Herstellung von 4-Toluidin bei der Nitrierung von Toluol startet, unter Bildung auch der Fehlisomere 2- und 3- Nitrotoluol, die nur in gewissem Maße destillativ abgetrennt werden, und anschließend das verbleibende, noch stärker verunreinigte 4-Nitrotoluol einer Hydrierung unterzogen wird.

In einer bevorzugten Ausführungsform wird das erfindungsgemäße Verfahren so durchgeführt, dass ein 4-Toluidin eingesetzt wird, welches 0,05 bis 0,19 Gew.%, besonders bevorzugt 0,08 bis 0,18 Gew.%, insbesondere 0,08 bis 0,15 Gew.% und insbesondere bevorzugt 0,08-0,12 Gew.% 3-Toluidin, bezogen auf 4-Toluidin enthält.

Bei der erfindungsgemäßen Umsetzung von 4-Toluidin mit dem angegebenen Maximal-Gehalt an 3-Toluidin mit mindestens, bevorzugt mehr als 2 mol Ethylen- oder Propylenoxid pro mol 4-Toluidin entsteht eine Mischung von verschiedenen alkoxylierten Derivaten des N,N-bis-(2-hydroxyethyl)-4-toluidins der allgemeinen Formel (I) mit verschiedenen Werten von n und m. Die Mischung kann dabei sowohl Verbindungen der allgemeinen Formel (I) enthalten, bei denen n und m im Molekül identisch sind, wie N,N-bis-(2-hydroxyethyl)-4-toluidin oder N,N-bis-(2-hydroxyethyloxyethylen)-4-toluidin, als auch Verbindungen der allgemeinen Formel (I), bei denen n und m im Molekül verschiedene Werte besitzen. Durch die Formulierung "Mischung von zwei oder mehr verschiedenen Verbindungen der allgemeinen Formel (I)" ist ausgeschlossen, dass ausschließlich eine Reinsubstanz wie N,N-Bis-(2-hydroxyethyl)-4-toluidin oder N,N-Bis-(2-hydroxyethyloxyethylen)-4-toluidin vorliegt.

Die Verteilungsbreite der erfindungsgemäßen Mischung kann im Hinblick auf den Alkoxylierungsgrad n + m der enthaltenen Verbindungen in engen Grenzen liegen oder aber auch breiter gestreut sein. Dies heißt zur Verdeutlichung: Wenn x mol Alkylenoxid pro mol 4-Toluidin eingesetzt werden, werden in der erfindungsgemäßen Mischung neben Verbindungen, die x mol Alkylenoxid aufgenommen haben, auch solche Verbindungen der allgemeinen Formel (I) gefunden, die beispielsweise x-2, x-1, x, x+1 oder x+2 mol Alkylenoxid aufgenommen haben. Die entsprechende Verteilung des Alkoxylierungsgrads läßt sich durch GC-MS ermitteln. Setzt man beispielsweise 3 mol Alkylenoxid pro mol 4-Toluidin ein, d.h. man geht von einem durchschnittlichen Alkoxylierungsgrad von 3 Molekülen Alkylenoxid pro Molekül 4-Toluidin aus, so kann die erfindungsgemäße Mischung auch Verbindungen der allgemeinen Formel (I) enthalten, die in Summe 1, 2, 3, 4, 5 und sogar 6 mol Alkylenoxid pro Molekül 4-Toluidin aufgenommen haben, d.h. Verbindungen, in denen die Summe aus n und m 1, 2, 3, 4, 5 oder 6 beträgt.

Die physikalischen Eigenschaften der erfindungsgemäßen Mischung von Verbindungen der allgemeinen Formel (I) können innerhalb bestimmter Grenzen durch den Alkoxylierungsgrad und die daraus resultierende Produktverteilung eingestellt und dem gewünschten Zweck angepasst werden. N,N-Bis-(2-hydroxyethyl)-4-toluidin ist ein Feststoff (Fp. 53-54°C), während die höher-alkoxylierten Derivate des 4-Toluidins bei Raumtemperatur flüssig sind. Je nach Breite der Verteilungskurve der unterschiedlich alkoxylierten Derivate kann also jeweils eine Mischung hergestellt werden, die für den jeweiligen technischen Einsatz gewünscht ist.

Derartige Mischungen von Verbindungen der allgemeinen Formel (I), die von einem speziellen 4-Toluidin ausgehen, sind in der Literatur bisher nirgends beschrieben.

Ethylen- und Propylenoxid können in kommerziell erhältlicher Form eingesetzt werden.

Das erfindungsgemäße Verfahren wird so durchgeführt, dass mindestens 2 mol, bevorzugt mehr als 2 mol, besonders bevorzugt 2,2 bis 5 mol, ganz besonders bevorzugt 2,3 bis 4 mol, insbesondere 2,3 bis 3,5 mol und insbesondere bevorzugt 2,5-2,6 mol Ethylenoxid oder Propylenoxid pro mol 4-Toluidin eingesetzt werden.

Die Durchführung des erfindungsgemäßen Verfahrens erfolgt so, dass man in eine Schmelze von 4-Toluidin bei erhöhter Temperatur das Alkylenoxid einleitet. Üblicherweise erfolgt die Reaktion bei einer Temperatur im Bereich von 100 bis 170°C, bevorzugt im Bereich von 110 bis 160°C, besonders bevorzugt im Bereich von 120 bis 150°C. In einer besonderen Ausführungsweise erfolgt die Reaktion in einer geschlossenen Apparatur unter leichtem Überdruck, wodurch ein Entweichen des Alkylenoxids in die Umwelt weitestgehend vermieden werden kann.

Das erfindungsgemäße Verfahren kann in Gegenwart eines Katalysators durchgeführt werden, es kann aber genauso ohne Katalysator gearbeitet werden. Als Katalysatoren können während der Alkoxylierung dem Fachmann prinzipiell geläufige basische Katalysatoren, bevorzugt NaOH, KOH oder NaOCH₃, eingesetzt werden. Der Katalysatoreinsatz ermöglicht ebenfalls eine Steuerung der Produktverteilung nach Wunsch und technischem Zweck. Einsetzbar ist der Katalysator in Mengen von bis zu 2 mol%, bevorzugt im Bereich von 0,05-1,0 mol%, bezogen auf das eingesetzte 4-Toluidin.

Die Verwendung von Ethylen- oder Propylenoxid als Alkoxylierungsmittel bietet gegenüber anderen Reagenzien (wie z.B. 2-Chlorethanol) den Vorteil, daß Hilfsreagenzien (wie stöchiometrische Mengen an Basen als HCl-Fänger) nicht eingesetzt werden müssen und dementsprechend keine Salze gebildet werden, die in einem gesonderten Schritt abgetrennt werden müßten.

Um das spezielle, besonders reine 4-Toluidin mit begrenztem 3-Toluidin-Gehalt zu erhalten, muß man aus dem technischen 4-Toluidin das 3-Toluidin durch sorgfältige Destillation entfernen, da die Siedepunkte der beiden Isomere nahe beinander liegen (Kp. von 4-Toluidin: 200,5°C; Kp. von 3-Toluidin: 203,4°).

Eine weitere Möglichkeit, 4-Toluidin mit einem Gehalt an 3-Toluidin von maximal 0,2 Gew.% herzustellen, besteht darin, technisches N-Acetyl-4-toluidin umzukristallisieren, anschließend zu verseifen und zu destillieren.

4-Toluidin mit einem Gehalt an 3-Toluidin von weniger als 0,2 Gew.% wird bevorzugterweise hergestellt, indem man zunächst Toluol nitriert, aus dem dabei entstehenden Isomerengemisch von 2-, 3- und 4-Nitrotoluol die beiden unerwünschten Isomere 2- und 3-Nitrotoluol durch Optimierung des Rücklaufverhältnisses sehr gut bis zur erforderlichen Reinheit destillativ abtrennt und das erhaltene 4-Nitrotoluol anschließend einer Hydrierung unter Bildung des 4-Toluidins gewünschter Reinheit unterwirft. Auf diesem Weg kann 4-Toluidin mit einem Gehalt von weniger als 0,2 Gew.%, bevorzugt von weniger als 0,1 Gew.% 3-Toluidin auch in technischen Mengen zur Verfügung gestellt werden.

Die Ausbeute der erfindungsgemäßen Alkoxylierung ist praktisch quantitativ und wird nur durch Verluste in der Handhabung begrenzt, wie sie z.B. beim Umfüllen durch Anhaften von Restmengen an der Reaktorwand entstehen.

Nach der beendeten Alkoxylierung hat es sich bewährt, die Reaktionsmischung auf eine Temperatur im Bereich von 60-100°C abzukühlen und für einen gewissen Zeitraum Stickstoff über die Reaktionsmischung zu leiten, um gegebenenfalls noch vorhandenes Alkylenoxid vollständig aus dem System zu entfernen.

Das Reaktionsgemisch kann nach dem Fachmann bekannten Methoden aufgearbeitet werden oder aber auch unmittelbar weiter verwendet werden.

Gegenstand der Erfindung ist ferner die Verwendung der erfindungsgemäßen Mischung von zwei oder mehr verschiedenen Verbindungen der allgemeinen Formel (I) als Polymerisations- oder Vulkanisationsbeschleuniger, bevorzugt in der radikalischen Polymerisation von Polyestern, insbesondere von ungesättigten Polyestern. Es hat sich dabei bewährt, die erfindungsgemäße Mischung in einer Menge von 0,1 - 5 Gew.% einzusetzen.

Die Verwendung solcher Mischungen anstelle von Reinsubstanzen ist vorteilhaft, da bei der Herstellung keinerlei Materialverluste z.B. durch Reinigung entstehen.

Wie bereits genannt, zeichnen sich die erfindungsgemäß hergestellten Mischungen dadurch aus, dass sie als Polymerisations- oder Vulkanisationsbeschleuniger bei der Herstellung farbloser Polymere eingesetzt werden können, da ihre Verwendung zu keinerlei Verfärbungen des Polymers führt. Dies ist je nach gewünschter Anwendung des Polymers von hoher Bedeutung und nicht mit beliebigen Polymerisations- und Vulkanisationsbeschleunigern des Standes der Technik erreichbar.

### Beispiele:

### Beispiel 1:

In einem 1-Ltr.-Glasautoklav mit Rührer, Innenthermometer, Einleitung für Ethylenoxid-Gas (Tauchrohr mit Fritte) und Steigrohr zum Ausnehmen legt man 321 g (3,0 mol) 4-Toluidin vor, das 0,1 Gew.% 3-Toluidin enthält. Man inertisiert, indem man dreimal evakuiert und jeweils wieder 0,5 bar Stickstoff aufdrückt. Man erhitzt - zunächst ohne Rühren - auf 100°C, um 4-Toluidin aufzuschmelzen (Fp. 43 °C).

Bei einem leichten inneren Überdruck an Stickstoff wird auf Verfahrenstemperatur von 120°C erwärmt und unter Rühren innerhalb von 10 Stunden 330 g (2,5 mol/mol 4-Toluidin) Ethylenoxid eingeleitet Nach jeweils 3 Stunden wird das Steigrohr kurz mit Stickstoff gespült, um weniger weit umgesetzte Anteile wieder unterzumischen.

Man rührt ca. 5 Stunden nach, kühlt auf 80 °C ab, entspannt und legt Vakuum an. Während dreistündigem Rühren entfernt man dabei Reste von Ethylenoxid.

Danach spült man noch mit Stickstoff und überführt den Autoklaveninhalt in eine Vorlage.

Die GC-Analyse ergibt, daß das Produkt kein 4-Toluidin mehr enthält (Nachweisgrenze: 100 ppm) und eine Mischung aus < 0,1 Flächen% N-Hydroxyethyl-4-toluidin, 50,1 Flächen% N,N-Bis-(hydroxyethyl)-4-toluidin sowie 43,7 Flächen% N-Oxyethyl-N-(hydroxyethyloxyethylen)-4-toluidin, 5,4 Flächen% isomerer Tetra- und 0,7 Flächen% isomerer Pentaoxethyl- und einer Spur Hexa-oxethylierter Isomerer darstellt.

### Beispiel 2:

In einem Autoklaven mit Rührer, Innenthermometer, eintauchendem Einleitungsrohr für das Ethylenoxid und Steigrohr zum Ausnehmen legt man 321 g (3,0 mol) 4-Toluidin vor, das 0,1 Gew.% 3-Toluidin enthält, gibt 1,0 g 30%ige Natrium-methanolat-Lösung zu und inertisiert mit Stickstoff.

Man erhitzt auf 100°C, um das 4-Toluidin aufzuschmelzen, startet den Rührer und leitet dann unter Rühren bei 120°C und einem Innendruck von 1400 mbar, ansteigend auf 2510 mbar innerhalb von 10 Stunden 340 g Ethylenoxid (2,58 mol pro mol 4-Toluidin) ein; nach jeweils 3 Stunden wird das Steigrohr freigeblasen, um eine gleichmäßige Umsetzung zu erreichen.

Man rührt zur vollständigen Reaktion noch 5 Stunden nach, kühlt auf 80°C ab und überführt den Inhalt des Autoklaven in eine Vorlage. Man erhält 630 g Produkt als leicht viskose, gelbliche, klare Flüssigkeit. Die GC-Analyse ergibt, dass das Produkt kein 4-Toluidin (Nachweisgrenze: 100 ppm) und kein N-Hydroxyethyl-4-toluidin mehr enthält; es enthält eine Mischung von 47,4 Flächen% N,N-Bis-(hydroxyethyl)-4-toluidin, 43,4 Flächen% N-Oxyethyl-N-(hydroxyethyloxyethyl)-4-toluidin sowie Spuren höher oxethylierter Verbindungen.

### Beispiel 3:

Man verfährt wie in Beispiel 2, leitet jedoch 400 g (3,0 mol/mol 4-Toluidin) Ethylenoxid ein; man erhält 690 g Produkt.

### Beispiel 4:

Man verfährt wie im Beispiel 2, führt die Reaktion aber bei 140°C durch. Erhalten werden 630 g Produkt.

### Beispiel 5:

Man geht vor wie in Beispiel 3, setzt jedoch KOH statt NaOH zu. Erhalten werden 690 g Produkt.

### Vergleichsbeispiel 6:

Man geht vor wie in Beispiel 2, verwendet jedoch technisches Toluidin mit einem Gehalt von 0,2 Gew.% 3-Toluidin. Erhalten werden 630 g Produkt.

### Beispiel 7:

Man geht vor wie in Beispiel 2, verwendet jedoch 4-Toluidin, das durch Acetylieren von technischem 4-Toluidin mit einem Gehalt an 3-Toluidin von 0,2 Gew.%, Umkristallisieren aus einem Gemisch aus 2-Propanol und Wasser, nachfolgendem Verseifen des N-Acetyl-4-toluidins und Destillation erhalten wird.

### Vergleichsbeispiel 8:

Man geht vor wie in Beispiel 2, setzt jedoch zur Ethoxylierung neben den 321 g (3,0 mol) technisch reinem 4-Toluidin, welches einen Gehalt von 0,1 Gew.% 3-Toluidin aufweist, zusätzlich 1 Gew. % 3-Toluidin bezogen auf das 4-Toluidin zu.

### Anwendungstest:

Die in den Beispielen 1-5 und 7 sowie in den Vergleichsbeispielen 6 und 8 hergestellten Mischungen werden im nachfolgenden Anwendungstest als Polymerisationsbeschleuniger verwendet. Hierzu wird ein ungesättigter Polyester, Roskydal^{®} K 14 M (Marke der BAYER AG) mit 0,5 Gew.% der jeweiligen Mischung versetzt und mit 3 Gew.%, bezogen auf den Polyester, Benzoylperoxyd polymerisiert.

Die Beurteilung der Färbung des erhaltenen Polymers erfolgt nach einer Skala von 0 bis 5; dabei bedeutet:
0: keine grüne Verfärbung
1: schwach grünlich
2. leicht grünlich
3: grünlich
4: starker grünlich
5: grasgrün

**Tabelle 1:**

| **Mischung aus Beispiel** | **Färbung des Polyesters nach Polymerisation in Gegenwart der Mischung Note** |
|---|---|
| 1 | 0 |
| 2 | 0 |
| 3 | 0 |
| 4 | 0 |
| 5 | 0 |
| Vergleichsbeispiel 6 | 4 |
| 7 | 0 |
| Vergleichsbeispiel 8 | 5 |

## Patentansprüche

1. Verfahren zur Herstellung einer Mischung von zwei oder mehr verschiedenen Verbindungen der allgemeinen Formel (I), 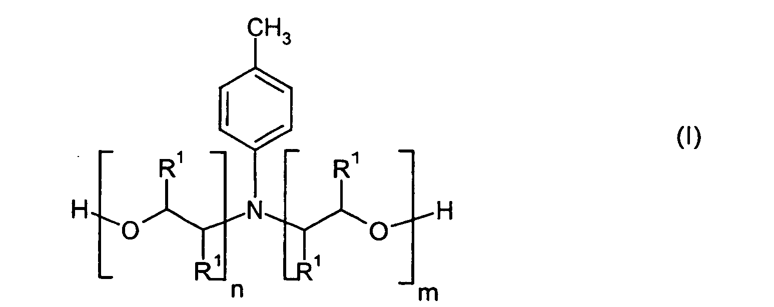 worin
n und m unabhängig voneinander eine ganze Zahl im Bereich von 0 bis 11 darstellen, die Summe aus n und m aber mindestens 1 beträgt und
R¹ gleich oder verschieden sind und Wasserstoff oder Methyl bedeuten können, wobei aber zwei Reste R¹, die sich an direkt benachbarten Kohlenstoffatomen befinden, nicht gleichzeitig Methyl darstellen,
durch Umsetzung von 4-Toluidin, welches weniger als 0,2 Gew.% 3-Toluidin, bezogen auf 4-Toluidin, enthält, mit mindestens 2 mol eines Alkylenoxids der Formel (II) pro mol 4-Toluidin 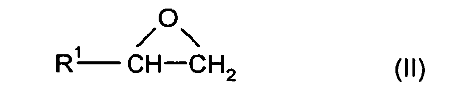 wobei R¹ die für die Formel (I) genannte Bedeutung besitzt

2. Verfahren nach Anspruch 1, **dadurch gekennzeichnet, dass** sowohl Verbindungen der allgemeinen Formel (I) erhalten werden, bei denen n und m im jeweiligen Molekül identisch sind, als auch Verbindungen der allgemeinen Formel (I), bei denen n und m im jeweiligen Molekül verschiedene Werte besitzen.

3. Verfahren nach Anspruch 1 oder 2, **dadurch gekennzeichnet, dass** 2,2 bis 5 mol, bevorzugt 2,3 bis 4 mol, besonders bevorzugt 2,3 bis 3,5 mol und insbesondere 2,5 bis 2.6 mol Alkylenoxid der allgemeinen Formel (II) pro mol 4-Toluidin eingesetzt werden.

4. Verfahren nach einem oder mehreren der Ansprüche 1 bis 3, **dadurch gekennzeichnet, dass** die Umsetzung bei einer Temperatur im Bereich von 100 bis 170°C, bevorzugt im Bereich von 110 bis 160°C, besonders bevorzugt im Bereich von 120 bis 150°C durchgeführt wird.

5. Verfahren nach einem oder mehreren der Ansprüche 1-4, **dadurch gekennzeichnet, dass** bei der Umsetzung basische Katalysatoren, bevorzugt NaOH, KOH oder NaOCH₃, eingesetzt werden.

6. Verfahren nach einem oder mehreren der Ansprüche 1-5, **dadurch gekennzeichnet, dass** ein 4-Toluidin eingesetzt wird, welches 0,05 bis 0,19 Gew.%, bevorzugt 0,08 bis 0,18 Gew.%, insbesondere 0,08 bis 0,15 Gew.% und insbesondere bevorzugt 0,08-0,12 Gew.% 3-Toluidin enthält.

7. Mischung von zwei oder mehr verschiedenen Verbindungen der allgemeinen Formel (I) erhältlich nach dem Verfahren gemäß einem oder mehreren der Ansprüche 1-6.

8. Verwendung der Mischung nach Anspruch 7 als Polymerisations- oder Vulkanisationsbeschleuniger, bevorzugt in der radikalischen Polymerisation von Polyestern, insbesondere ungesättigten Polyestern.

9. Polymeres Produkt erhältlich durch radikalische Polymerisation eines Polyesters, bevorzugt eines ungesättigten Polyesters, in Gegenwart einer Mischung nach Anspruch 7 als Polymerisations- oder Vulkanisationsbeschleuniger.

## Claims

1. Process for preparing a mixture of two or more different compounds of the general formula (I) 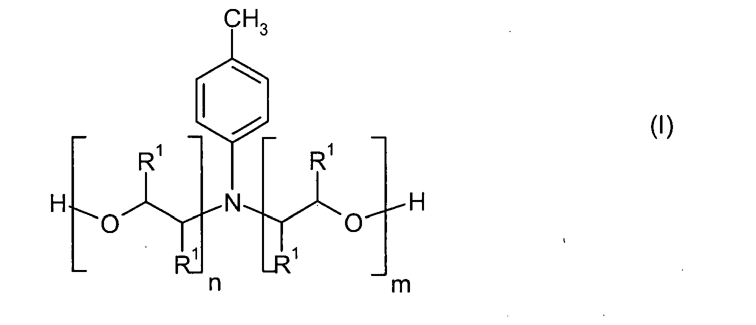 in which
n and m independently of one another are each an integer in the range from 0 to 11, but the sum of n and m is at least 1, and
R¹ radicals are identical or different and can be hydrogen or methyl, although two radicals R¹ located on directly adjacent carbon atoms are not simultaneously methyl,
by reacting 4-toluidine, containing less than 0.2% by weight of 3-toluidine, based on 4-toluidine, with at least 2 mol of an alkylene oxide of the formula (II) per mole of 4-toluidine 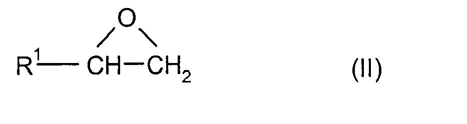 where R¹ possesses the definition specified for the formula (I).

2. Process according to Claim 1, **characterized in that** not only compounds of the general formula (I) in which n and m are identical in the respective molecule but also compounds of the general formula (I) in which n and m possess different values in the respective molecule are obtained.

3. Process according to Claim 1 or 2, **characterized in that** 2.2 to 5 mol, preferably 2.3 to 4 mol, more preferably 2.3 to 3.5 mol and in particular 2.5 to 2.6 mol of alkylene oxide of the general formula (II) are used per mole of 4-toluidine.

4. Process according to one or more of Claims 1 to 3, **characterized in that** the reaction is carried out at a temperature in the range from 100 to 170°C, preferably in the range from 110 to 160°C, more preferably in the range from 120 to 150°C.

5. Process according to one or more of Claims 1-4, **characterized in that** the reaction is carried out using basic catalysts, preferably NaOH, KOH or NaOCH₃.

6. Process according to one or more of Claims 1-5, **characterized in that** a 4-toluidine is used which contains 0.05% to 0.19%, preferably 0.08% to 0.18%, in particular 0.08% to 0.15% and with particular preference 0.08%-0.12% by weight of 3-toluidine.

7. Mixture of two or more different compounds of the general formula (I) obtainable by the process according to one or more of Claims 1-6.

8. Use of the mixture according to Claim 7 as a polymerization accelerator or vulcanization accelerator, preferably in the free-radical addition polymerization of polyesters, especially unsaturated polyesters.

9. Polymeric product obtainable by free-radical addition polymerization of a polyester, preferably of an unsaturated polyester, in the presence of a mixture according to Claim 7 as polymerization regulator or vulcanization regulator.

## Revendications

1. Procédé de préparation d'un mélange de deux composés différents ou plus de formule générale (I), 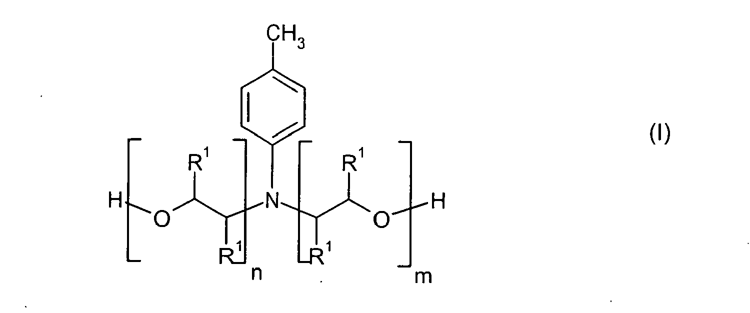 dans laquelle :
n et m représentent, indépendamment l'un de l'autre, un entier dans la plage de 0 à 11, mais la somme de n et m vaut au moins 1, et
R¹ est identique ou différent et peut représenter un atome d'hydrogène ou un groupe méthyle, mais deux radicaux R¹, qui se trouvent sur des atomes de carbone directement voisins, ne représentent pas simultanément un groupe méthyle,
par réaction de la 4-toluidine, qui contient moins de 0,2 % en poids de 3-toluidine, par rapport à la 4-toluidine, avec au moins 2 mol d'un oxyde d'alkylène de formule (II) par mole de 4-toluidine 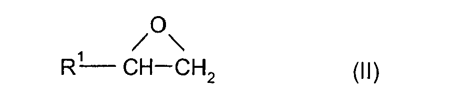 dans laquelle R¹ présente la définition mentionnée pour la formule (1).

2. Procédé selon la revendication 1, **caractérisé en ce que** l'on obtient aussi bien des composés de formule générale (I), dans laquelle n et m sont identiques dans chaque molécule, que des composés de formule générale (I), dans laquelle n et m présentent des valeurs différentes dans chaque molécule.

3. Procédé selon la revendication 1 ou 2, **caractérisé en ce que** de 2,2 à 5 mol, de préférence de 2,3 à 4 mol, particulièrement préférablement de 2,3 à 3,5 mol et, en particulier, de 2,5 à 2,6 mol d'oxyde d'alkylène de formule générale (II), sont utilisées par mole de 4-toluidine.

4. Procédé selon l'une ou plusieurs des revendications 1 à 3, **caractérisé en ce que** la réaction est effectuée à une température dans la plage de 100 à 170 °C, de préférence dans la plage de 110 à 160°C, particulièrement préférablement dans la plage de 120 à 150 °C.

5. Procédé selon l'une ou plusieurs des revendications 1 à 4, **caractérisé en ce que** l'on utilise des catalyseurs basiques, de préférence, du NaOH, du KOH ou du NaOCH₃, lors de la réaction.

6. Procédé selon l'une ou plusieurs des revendications 1 à 5, **caractérisé en ce que** l'on utilise une 4-toluidine qui contient de 0,05 % à 0,19 % en poids, de préférence de 0,08 % à 0,18 % en poids, en particulier de 0,08 % à 0,15 % en poids, et particulièrement préférablement de 0,08 % à 0,12 % en poids, de 3-toluidine.

7. Mélange de deux composés différents ou plus de formule générale (I) pouvant être obtenus conformément au procédé selon l'une ou plusieurs des revendications 1 à 6.

8. Utilisation du mélange selon la revendication 7, en tant qu'accélérateur de polymérisation ou de vulcanisation, de préférence lors de la polymérisation radicalaire de polyesters, en particulier de polyesters insaturés.

9. Produit polymère pouvant être obtenu par la polymérisation radicalaire d'un polyester, de préférence d'un polyester insaturé, en présence d'un mélange selon la revendication 7, en tant qu'accélérateur de polymérisation ou de vulcanisation.
